# EUROPEAN PATENT SPECIFICATION

(11) **EP 4 382 118 B1**
(45) Date of publication and mention of the grant of the patent: **20.08.2025**
(21) Application number: 22211973.7
(22) Date of filing: 07.12.2022
(51) Int. Cl.: A61K 40/11, A61K 40/31, A61K 40/42, C12N 15/113

(54) **MODIFIED IMMUNE CELL**
MODIFIZIERTE IMMUNZELLE
CELLULE IMMUNITAIRE MODIFIÉE

(43) Date of publication of application: 12.06.2024
(73) Proprietor: Eberhard Karls Universität Tübingen, Medizinische Fakultät, 72074 Tübingen (DE)
(72) Inventor: Bucher, Philip, 72070 Tübingen (DE); Grimm, Melanie, 72135 Dettenhausen (DE); Feucht, Judith, 72076 Tübingen (DE); Leibold, Josef, 72076 Tübingen (DE)
(74) Representative: Witte, Weller & Partner Patentanwälte mbB

(56) References cited:
- WO-A1-2021/158850
- TAKEDA ANDREW J. ET AL: "Novel PIK3CD mutations affecting N-terminal residues of p110[delta] cause activated PI3K[delta] syndrome (APDS) in humans", JOURNAL OF ALLERGY AND CLINICAL IMMUNOLOGY, vol. 140, no. 4, 1 October 2017 (2017-10-01), AMSTERDAM, NL, pages 1152 - 1156.e10, XP093042071, ISSN: 0091-6749, DOI: 10.1016/j.jaci.2017.03.026
- FUNK CHRISTOPHER RONALD ET AL: "PI3K[delta]/[gamma] inhibition promotes human CART cell epigenetic and metabolic reprogramming to enhance antitumor cytotoxicity", BLOOD, AMERICAN SOCIETY OF HEMATOLOGY, US, vol. 139, no. 4, 14 September 2021 (2021-09-14), pages 523 - 537, XP086942054, ISSN: 0006-4971, [retrieved on 20210914], DOI: 10.1182/BLOOD.2021011597
- JIA YANJUN ET AL: "Hyperactive PI3K[delta] predisposes naive T cells to activation via aerobic glycolysis programs", CELLULAR & MOLECULAR IMMUNOLOGY, NATURE PUBLISHING GROUP UK, LONDON, vol. 18, no. 7, 25 February 2020 (2020-02-25), pages 1783 - 1797, XP037495066, ISSN: 1672-7681, [retrieved on 20200225], DOI: 10.1038/S41423-020-0379-X
- JAFARZADEH LEILA ET AL: "Prolonged Persistence of Chimeric Antigen Receptor (CAR) T Cell in Adoptive Cancer Immunotherapy: Challenges and Ways Forward", FRONTIERS IN IMMUNOLOGY, vol. 11, 1 January 2020 (2020-01-01), pages 70200702, XP055857812, DOI: 10.3389/fimmu.2020.00702

## Description

The present invention relates to a chimeric antigen receptor (CAR) T or NK cell, and to a method for preparing such CAR T or NK cell.

### BACKGROUND OF THE INVENTION

Cellular immunotherapy, also known as adoptive cell therapy, is a form of treatment that uses the cells of or derived from the immune system to combat cancer or non-malignant diseases. Currently, cellular immunotherapy can be classified into three different types with each their own mechanism of action, namely cellular immunotherapy with tumor-infiltrating lymphocytes (TIL), cellular immunotherapy using T cell receptor (TCR) gene therapy, and cellular immunotherapy with chimeric antigen receptor (CAR) modified T cells. The use of other immune cell types such as natural killer (NK) cells as a basis for cell therapy is also an area of current research.

CAR T cells are T cells that have been genetically engineered to express a synthetic receptor. Chimeric antigen receptors (CARs, also known as chimeric antigen receptors, chimeric immunoreceptors, or artificial T cell receptors) are receptor proteins that have been introduced into immune cells to give them the new ability to target a specific antigen. The receptors are chimeric because they combine both antigen-binding domains that are most commonly derived from antibodies and T cell activating functions into a single receptor.

CAR T cell therapy uses T cells engineered with CARs for therapy, predominantly against cancer. The premise of CAR T immunotherapy is to modify T cells to recognize cancer cells in order to more effectively target and destroy them. Scientists harvest T cells from people, genetically alter them, then infuse the resulting CAR T cells into patients to attack their tumors. CAR T cells can be both CD4⁺ and CD8⁺, typically with a 1-to-1 ratio of both cell types providing synergistic antitumor effects.

CAR T cells can be either derived from T cells in a patient's own blood (autologous) or derived from the T cells of another healthy donor (allogeneic). Once isolated from a person, these T cells are genetically engineered to express a specific CAR, which reprograms them to target an antigen that is present on the surface of tumors. For safety, CAR T cells are most commonly engineered to be specific to an antigen expressed on a tumor that is not highly expressed on healthy cells.

After CAR T cells are infused into a patient, they act as a "living drug" against cancer cells. When they come in contact with their targeted antigen on a cell, CAR T cells bind to it and become activated so that they proliferate and become cytotoxic. CAR T cells destroy cells through several mechanisms, including extensive stimulated cell proliferation, thereby increasing the effector to target ratio and cytotoxicity capacity to other living cells (cytotoxicity) and increased secretion of factors that can affect other cells such as cytokines, chemokines, interleukins and growth factors. The first CAR T cell therapies were approved by the FDA in 2017. Based on successful clinical results, CAR T cell therapies have been approved against various leukemias, lymphomas and multiple myeloma in recent years.

The CAR itself consists of an extracellular antigen binding domain, often a single chain variable fragment (scFv) of an antibody, a hinge and transmembrane domain and an intracellular domain, usually a co-stimulatory domain and part of the CD3ζ protein. Binding of the extracellular antigen-binding domain, e.g., the scFv of an anti-CD19 antibody to CD19, activates CAR T cells via the intracellular co-stimulatory and the CD3ζ signal transduction domains, resulting in cytotoxicity of CAR T cells towards CD19-positive cells. Since many B-cell lymphomas and leukemias express CD19 on their surface, anti-CD19 CAR T cells can be used to target these tumors.

CARs with a 4-1BB co-stimulatory domain and a CD3ζ signal transduction domain ("BBz") directed against the antigen CD19 ("19BBz"), for example, have shown good long-term persistence in patients; see Melenhorst, J. J. et al. (2022), Decade-long leukaemia remissions with persistence of CD4+ CAR T cells. Nature 602, 503-509, doi:10.1038/s41586-021-04390-6. The 4-1BB-derived co-stimulatory domain activates the NFκB signaling pathway via TRAF2. In addition, CARs with the BBz signaling domain have been shown to induce decreased T cell differentiation and exhaustion and to proliferate better *ex vivo*; see Kawalekar, O. U. et al. (2016), Distinct Signaling of Coreceptors Regulates Specific Metabolism Pathways and Impacts Memory Development in CAR T Cells. Immunity 44, 712, doi:https://doi.org/10.1016/j.immuni.2016.02.023. In contrast, the 28z domain imparts higher effector function on T cells and provides improved antigen sensitivity, but can induce enhanced tonic signaling; Long, A. H. et al. (2015), 4-1BB costimulation ameliorates T cell exhaustion induced by tonic signaling of chimeric antigen receptors. Nat Med 21, 581-590, doi:10.1038/nm.3838.

CAR T cell therapy shows an initial response of about 90% in B-subtype acute lymphoblastic leukemia (B-ALL) patients. However, not all tumors respond comparably well, and recurrences may occur during the course. Differences in antigen density and immunoregulatory factors influence CAR T cell activity and functionality. For example, CAR T cells have been shown to have reduced antigen sensitivity to target cells with low antigen expression. This reduced antigen sensitivity poses a challenge, for example, in solid tumors with heterogeneous antigen expression as well as in relapses after CAR T cell therapy, which may be associated with downregulation of the target antigen on the tumor cell. In addition, some CAR T cells show no sustained functionality and/or limited proliferation, resulting in tumor progression or recurrence.

Takeda et al. (2017), J. Allergy Clin. Immunol., Vol. 140, pp. 1152-1156, discloses cells obtained from a patient with an E81K mutation in the PIK3CD gene with increased basal activity. It also discloses T cells modified to overexpress E81K resulting in an activated PI3K pathway.

WO 2021/158850 discloses CD19 CAR T cells with CD28 and CD3 as intracellular domains. CD28 as part of the CAR activates PI3K signaling by binding to p85. The document discloses said CARs and different mutations in the CD28 YMNM motif.

Funk et al. (2021), Blood, Vol. 139, pp. 523-537, discloses anti-CD19 CAR T cells with CD28 and CD3 intracellular domains which were treated with a PI3Kd/g inhibitor (Duvelisib) resulting in reduced PI3K levels.

Jia et al. (2020), Cellular & Molecular Immunology, Vol. 18, pp. 1783-1797, discloses a CRISPR knock-in mouse introducing a E1024K mutation in the PIK3CD gene resulting in increased PI3K/AKT signaling in T cells.

Jafarzadeh et al. (2020), Frontiers in Immunology, Vol. 11:702, reviews inhibition of the PI3K pathway to improve CAR T cell functions by employing inhibitors of said pathway.

Against this background, the invention is thus based on the problem of further improving the activity of cellular immunotherapies and increasing their functionality.

### SUMMARY OF THE INVENTION

The problem underlying the invention is solved by the provision of a chimeric antigen receptor (CAR) T or NK cell characterized by a modified phosphoinositide 3-kinase (PI3K) pathway compared to a non-modified reference immune cell, which CAR T or NK cell comprises a modified PI3K characterized by increased activity compared to a wild type PI3K, wherein said modified PI3K comprises a point mutation.

The problem is also solved by a method for preparing a chimeric antigen receptor (CAR) T or NK cell characterized by a modified PI3K pathway compared to a non-modified reference immune cell, comprising (i) providing a CAR T or NK cell, and (ii) introducing a point mutation into PI3K comprised by the CAR T or NK cell such that its activity is modified compared to wild type PI3K.

According to the invention, an "immune cell" or, synonymously "immunoresponsive cell" can be a cell of the lymphoid lineage. The lymphoid lineage, comprising B, T and natural killer (NK) cells, provides for the production of antibodies, regulation of the cellular immune system, detection of foreign agents in the blood, detection of cells foreign to the host, and the like. Non-limiting examples of immune cells of the lymphoid lineage include T cells, natural killer (NK) cells, embryonic stem cells, and pluripotent stem cells (e.g., those from which lymphoid cells may be differentiated). T cells can be lymphocytes that mature in the thymus and are chiefly responsible for cell-mediated immunity. T cells are involved in the adaptive immune system. The T cells of the presently disclosed subject matter can be any type of T cells, including, but not limited to, helper T cells, cytotoxic T cells, memory T cells (including central memory T cells, stem-cell-like memory T cells (or stem-like memory T cells), and two types of effector memory T cells: e.g., TEM cells and TEMRA cells, regulatory T cells (also known as suppressor T cells), natural killer T cells, mucosal associated invariant T cells, and γδ T cells. Cytotoxic T cells (CTL or killer T cells) are a subset of T lymphocytes capable of inducing the death of infected somatic or tumor cells. A patient's own T cells may be genetically modified to target specific antigens through the introduction of a CAR. The T cell can be a CD4⁺ T cell or a CD8⁺ T cell. In certain embodiments, the T cell is a CD4⁺ T cell. In certain embodiments, the T cell is a CD8⁺ T cell. Natural killer (NK) cells can be lymphocytes that are part of cell-mediated immunity and act during the innate immune response. NK cells do not require prior activation in order to perform their cytotoxic effect on target cells.

According to the invention, the CAR T or NK cell is in particular a mammalian cell, including a human cell.

The immune cell may be "isolated". By "isolated immune cell" is meant an immune cell that is separated from the molecular and/or cellular components that naturally accompany the cell. The terms "isolated", "purified", or "biologically pure", which can be used interchangeably, refer to material that is free to varying degrees from components which normally accompany it as found in its native state. "Isolate" denotes a degree of separation from original source or surroundings. A "purified" or "biologically pure" cell is sufficiently free of other materials such that any impurities do not materially affect the biological properties of the cell or cause other adverse consequences. Purity and homogeneity are typically determined using analytical chemistry techniques, for example, FACS analysis, polyacrylamide gel electrophoresis or high-performance liquid chromatography.

The "PI3K" (phosphoinositide 3-kinase) or "PI3K/AKT/mTOR" pathway is an intracellular signaling pathway which is important in regulating the cell cycle. It is directly related to cellular quiescence, proliferation, cancer, and longevity. PI3K activation phosphorylates and activates AKT, localizing it in the plasma membrane. AKT can have a number of downstream effects such as activating CREB, inhibiting p27, localizing FOXO in the cytoplasm, and activating mTOR which can affect transcription of p70 or 4EBP1. An overview of the PI3K signaling pathway is given in Hemmings and Restuccia (2015), PI3K-PKB/Akt Pathway, Cold Spring Harb Perspect Biol.; 7(4): a026609.

At the center of the PI3K signaling pathway is the phosphatidylinositol 3-kinase (PI3K) which constitutes a family of related intracellular signal transducer enzymes capable of phosphorylating the 3 position hydroxyl group of the inositol ring of phosphatidylinositol (Ptdlns). Mammals have eight isoforms of PI3K, divided into four classes: Class I, Class II, Class III, and Class IV. Enzymes of all three classes and all isoforms are included according to the invention, in particular the delta isoform PI3Kδ that in humans is encoded by the PIK3CD gene.

The term "modified" means that in the immune cell, the PI3K pathway is or has been altered compared to a reference immune cell. The modification results in increased activity of the signaling pathway.

The modification of the PI3K pathway can be realized by various methods well known to the skilled artisan. They include the targeted modification of the phosphatidylinositol 3-kinase (PI3K or PI3Kδ). Such targeted modification can be achieved by, e.g., CRISPR/Cas9 and homology directed repair (HDR), CRISPR/Cas9 based systems like prime or base editing, transposon-based systems to overexpress the cDNA containing the respective modification (e.g., mutation), viral overexpression of such a cDNA, TALEN/zinc-finger-based approaches, etc.

By "reference" or, synonymously, "control," is meant a standard of comparison. Thus, "reference immune cell" means such an immune cell in which the PI3K pathway is unchanged and therefore can illustrate the modification of the signaling pathway in the immune cell of the invention.

The inventors have recognized that the CAR T or NK cell according to the invention can be given improved activity and increased functionality by the modified PI3K pathway. The CAR T or NK cell according to the invention thus acquires therapeutic potential and is particularly well suited for use in immune cell therapy, especially also against target cells with low antigen expression.

In an embodiment of the invention said CAR T or NK cell is characterized by an overactive PI3K pathway compared to a non-modified reference immune cell.

The inventors have found that an increase in the PI3K signaling pathway in the CAR T or NK cell of the invention significantly improves its activity and therapeutic suitability. Such CAR T or NK cells with overactive PI3K signaling pathway have been found to be particularly suitable for use against pathogenic structures (e.g., tumor cells) with high, medium and also with low antigen levels or low antigen expression. They are characterized by markedly enhanced cytotoxicity and reduced exhaustion and differentiation, as well as prolonged persistence.

This finding was not expected, since a large number of tumors in particular are characterized by an overactive PI3K signaling pathway. Surprisingly, the inventors have succeeded in re-functioning a pathological, tumor-specific overactive signaling pathway, which could otherwise have fatal consequences, and in using it therapeutically, particularly against tumor diseases.

In the invention the CAR T or NK cell comprises a modified phosphoinositide 3-kinase (PI3K) characterized by increased activity compared to a non-modified reference PI3K.

This measure advantageously modifies the key enzyme of the PI3K signaling pathway in such a way that this results in an increased activity of the entire PI3K pathway. The modification of PI3K can be realized in ways known to the skilled person, e.g., by targeted mutagenesis, genomic editing, etc. As mentioned above, PI3K enzymes of all three classes and all isoforms are envisaged according to the invention, in particular the delta isoform PI3Kδ.

In the invention said modified PI3K comprises a point mutation, preferably at amino acid position 81, further preferably by said point mutation in PI3K a glutamic acid (E) at position 81 is replaced by another amino acid, preferably by a lysine (K) (PI3K^{E81K}).

Typically, the basal activity of the PI3K^{E81K} enzyme is increased *in vitro* only about 10-fold compared to the wild type, whereas the activated enzyme shows an activation of more than 200-fold. The inventors found that via such a point mutation, an overactive PI3K and an activity-enhanced downstream signaling pathway can be created in a long-term stable manner. This was particularly surprising. Indeed, in the prior art, such a PI3K mutation was described in a few patients, but there it led to an increased proportion of CD57⁺ T cells and an increased induction of T cell exhaustion during the course; see Takeda et al. (2018), Novel PIK3CD mutations affecting N-terminal residues of p110δ cause APDS1 in humans, J Allergy Clin Immunol, 140(4): 1152-1156.e10. In contrast, the inventors were not able to observe these phenomena in the CAR T or NK cell of the invention, even after multiple rounds of antigen stimulations, but were able to demonstrate an increased functionality of the E81K-mutated immune cells that was also sustained over several weeks.

Another advantage of the PI3K mutation is that the inventors show no transforming effects. The inventors studied cell growth without and with cytokines and with and without antigen stimulation. This showed no increased cell proliferation of E81K-mutated cells compared with wild-type cells in an antigen-independent context. The E81K mutant cells showed increased cell growth only under antigen stimulation, indicating an enhanced specific proliferation potential. Therefore, the E81K mutation is not associated with malignant potential. Furthermore, the E81K mutation has been detected in tumor patients in only four cases out of a cohort of over 64,000 cases, and no T-cell tumor is represented. The lack of any malignant potential is also confirmed by the fact that patients described in the literature with this mutation did not develop T-cell malignancy; cf. Takeda et al. (op. cit.). The modified CAR T or NK cell according to the invention is therefore characterized by a very good safety profile.

In particular, the E81K point mutation can be provided in the delta form of the enzyme, i.e., PI3Kδ^{E81K}.

Te invention provides such a modified immune cell that is particularly suitable for immune cell therapies. T cells in particular are of interest here, as they represent the typical starting material for chimeric antigen receptor (CAR) T cells. However, CAR NK cell therapy is also becoming increasingly important.

Therefore, the invention provides a chimeric antigen receptor (CAR) T or NK cell, which CAR comprises an extracellular antigen-binding domain, a transmembrane domain, and an intracellular signaling domain.

According to the invention, CAR T cell is provided, this means that the immune cell has been genetically engineered to express a synthetic receptor, which comprises an extracellular antigen-binding domain, a transmembrane domain, and an intracellular signaling domain, as outlined above. It may also comprise a hinge/spacer domain. The receptor is chimeric because it combines both antigen-binding domains which are most commonly derived from antibodies, and T cell activating functions into a single receptor. It links an extracellular antigen binding/recognition domain via a hinge and transmembrane domain to an intracellular signaling domain, which activates the T cell when an antigen is bound. The antigen binding/recognition domain is exposed to the outside of the cell, in the ectodomain portion of the receptor. It interacts with potential target molecules and is responsible for targeting the CAR T cell to any cell expressing a matching molecule. The antigen binding/recognition domain is typically, but not exclusively, derived from the variable regions of a monoclonal antibody linked together as a single-chain variable fragment (scFv). The transmembrane domain is a structural component, typically consisting of a hydrophobic alpha helix that spans at least a portion of the cell membrane. It anchors the CAR to the plasma membrane, bridging the extracellular antigen binding/recognition domains with the intracellular signaling region. This domain is essential for the stability of the receptor as a whole. The intracellular T cell signaling domain lies in the receptor's endodomain, inside the cell. After an antigen is bound to the external antigen recognition domain, CAR receptors cluster together and transmit an activation signal. Then the internal cytoplasmic end of the receptor perpetuates signaling inside the T cell.

In principle all CAR constructs can be used in combination with modified PI3K pathway.

The antigens can be chosen arbitrarily, depending on the field of application and the purpose of the cell. Possible antigens include, but are not limited to CD19, ADGRE2, BCMA, CAIX, CEA, CCR1, CCR4, CD5, CD3, CD8, CD7, CD10, CD20, CD22, CD30, CLL1, CD33, CD34, CD38, CD41, CD44, CD44V6, CD49f, CD56, CD70, CD74, CD79b, CD87 (uPAR), CD99, CD123, CD133, CD138, CD276 (B7H3), CLEC12A, EGP-2, EGP-40, EphA2, EpCAM, erb-B2,3,4, ERBB, EGF1R, EGFR-VIII, FBP, fetal acetylcholine receptor, folate receptor-a, GD2, GD3, GP100, HER-2, hTERT, Integrin B7, ICAM-1, IL-13R-a2, K-light chain, KDR, LeY, L1 cell adhesion molecule (L1CAM), LILRB2, MAGE-A1, Mesothelin, MUC16, MUC1, MAGEA3, p53, MART1, NKCS1, NY-ESO, Proteinase3 (PR1), Tyrosinase, Survivin, hTERT, NKG2D ligands, -1, oncofetal antigen (h5T4), PRAME, PSCA, PSMA, RAS oncogenes (G12V, Q61 H/L/R), ROR1, TAG-72, TP53 (R175H), TRBC1, TRBC2, TIM-3, VEGF-R2, WT-1.

The transmembrane domain (as well as a hinge/spacer region) of the CAR can be comprised of a native or modified domain or combination of CD4, CD8, CD8a, CD8b, ICOS, CD27, NKGD2, CD28, CD3z, 4-1BB, OX-40, CD40 but are not limited to those domains.

The intracellular signaling and/or co-stimulatory domain can comprise various polypeptides such as CD3ζ, CD137 (4-1BB), CD28, 4-1BB, OX40, ICOS, CD27, DAP-10, CD40, NKGD2, ICOS peptide or a combination thereof and are not limited to this selection.

With this measure, the invention eliminates or at least reduces the disadvantages of the currently implemented CAR T cell therapies. In particular, tumor patients, e.g., patients with a B-form of acute lymphoblastic leukemia (B-ALL) or lymphoma, often experience recurrences in the later course of therapy despite a high initial response to the therapy. Diverse differences in antigen density and immunoregulatory factors influence CAR T cell activity and functionality. For example, current CAR T cells have been shown to have reduced antigen sensitivity to target cells with low antigen expression. In addition, some known CAR T cells do not exhibit sustained functionality and/or limited proliferation, resulting in tumor progression or recurrence. The invention remedies this situation. In the CAR T cell of the invention, the activated modified PI3K enzyme is more active than in a reference cell with non-modified Pi3K enzyme. The overactive PI3K pathway enhances the cytotoxic activity of, e.g., BBz CAR T cells while maintaining the beneficial properties of reduced T cell exhaustion and differentiation and prolonged persistence. Furthermore, it has been shown that enhanced PI3K signaling in CAR T cells is associated with improved activity against tumors with low antigen expression, so PI3K modification may lead to increased efficacy against tumor cells with low antigen expression in different CAR designs.

In still another embodiment of the invention the CAR's intracellular signaling domain comprises a CD3ζ polypeptide or modifications thereof, while in another or the same embodiment the intracellular signaling domain comprises a CD137 (4-1BB) or CD28 costimulatory polypeptide. In still another or the same embodiment the extracellular antigen-binding domain comprises an CD19 binding polypeptide.

By this measure, the invention can be applied to CAR T cells, which are currently the most commonly used therapeutic cells.

CD3ζ is a homodimer-forming type 1 transmembrane (TM) protein and is part of the T-cell receptor complex (TCR-CD3). CD3ζ possesses a long cytoplasmic part that contains three immunoreceptor tyrosine-based activation motifs (ITAMs), which correspond to the six tyrosines that get phosphorylated upon antigen binding to the extracellular part of TCRαβ. Phosphorylation subsequently activates several downstream signaling cascades. To mimic this process, CD3ζ's cytoplasmic domain is commonly used as the main CAR endodomain component. The intracellular domain can also comprise modifications of CD3ζ, such as a modified CD3ζ polypeptide comprising or consisting of one, two or three ITAMs (e.g., anti-CD19 CARs SFG-1928ζ, SFG-1928ζ-1xx, SFG-1928ζ-xx3).

CD137 (4-1BB; TNFSR9) is an activation-induced surface receptor that through costimulation effects provides antigen-primed T cells with augmented survival, proliferation and effector functions as well as metabolic advantages. It is an important regulator of immune responses.

CD28 (Tp44) is one of the proteins expressed on T cells that provide co-stimulatory signals required for T cell activation and survival. T cell stimulation through CD28 in addition to the T-cell receptor (TCR) can provide a potent signal for the production of various interleukins (IL-6 in particular).

CD19 (cluster of differentiation 19), also known as B-lymphocyte antigen CD19 or B-Lymphocyte Surface Antigen B4, T-Cell Surface Antigen Leu-12 and CVID3 is a transmembrane protein that in humans is encoded by the gene CD19. In principle, any T cell and other immune cells can be genetically modified and the modified CAR therapy can be directed against any surface molecule. However, the CD19 receptor is often preferred because it is very common in B-cell leukemias and B-cell lymphomas and is typically higher and more frequently expressed compared to other surface molecules, such as CD20 or CD22. Since the B-lymphocyte antigen CD19 is found exclusively on B cells, adverse "on-target" effects are rare and usually well treatable.

By this measure, the immune cell according to the invention is adapted to the currently very commonly used CAR-T cells and improves them in their activity and functionality.

According to the invention, a "polypeptide" refers to a chain of amino acids linked by peptide bonds of at least 10 or more amino acids. In order to speak of a CD3ζ-, CD137- or CD19 polypeptide according to the invention, the polypeptide must have at least specific activity of the molecules mentioned above. A polypeptide that contains more than approximately 50 amino acids is known as a protein. According to the invention, a "polypeptide" also includes a protein, be it a full-length protein or a functional fragment thereof.

In another embodiment of the invention the PI3K is modified by genome editing.

Editing of PI3K in the cell of the invention at the genomic level is particularly suitable. This ensures that - unlike viral transductions - there is no exogenous overexpression of PI3Kδ and no uncoupling of the biological receptor. In this way, negative regulation is also ensured. At the same time, however, good efficiency and feasibility in primary immune cells is ensured.

A genomic editing can be achieved by, e.g., CRISPR/Cas9 and homology directed repair (HDR), CRISPR/Cas9 based systems like prime or base editing, transposon-based systems to overexpress the cDNA containing the respective modification (e.g., mutation), viral overexpression of such a cDNA, TALEN/zinc-finger-based approaches, etc.

In one embodiment of the invention, editing at the genomic level is performed by the CRISPR/Cas system, preferably a CRISPR/Cas9 system. In particular, this allows the targeted and safe introduction of the above-mentioned point mutation, e.g., E81K, into the PI3K enzyme.

In certain embodiments, a CRISPR/Cas9 variant is used, which consists of a Cas9 nickase and causes only a single-strand break. This has the major advantage that the formation of translocations as well as the DNA damage response caused by any double strand break is significantly reduced. In one embodiment, the Cas9 nickase can also be coupled to a cytidine deaminase and possibly two uracil DNA glycosylase inhibitors (UGIs), which together constitute the so-called base editor. The binding of the sgRNA to the complementary DNA sequence and the base editor results in a single-strand break at the target sequence as well as in the transcription of the cytidine to a thymine.

In another embodiment of the invention in the CRISPR/Cas9 editing a sgRNA molecule is used comprising the nucleotide sequence of gctcttgctgctccgctgtc (SEQ ID NO: 1) and/or aagagctggaggacgagcaa (SEQ ID NO: 2).

This measure employs an sgRNA molecule that the inventors found to be well suitable for targeted genomic editing and, in particular, the introduction of the PI3K^{E81K} point mutation.

Therefore, another subject-matter of the disclosure is an oligonucleotide, preferably a sgRNA molecule, comprising the nucleotide sequence of gctcttgctgctccgctgtc (SEQ ID NO: 1) and/or aagagctggaggacgagcaa (SEQ ID NO: 2), and the use of said oligonucleotide for genome editing the phosphoinositide 3-kinase (PI3K) gene in an immune cell.

Against this background another subject-matter of the disclosure is an isolated modified immune cell prepared by the method according to the invention.

A still further subject-matter of the invention is the isolated modified immune cell according to the invention for use in medicine. Such a use includes tumor diseases or cancer and non-malignant diseases such as autoimmune diseases. In another embodiment the diseases are cancers, such as leukemias and/or lymphomas.

Still another subject-matter of the disclosure is a pharmaceutical composition comprising an effective amount of the CAR T or NKcell according to the invention and a pharmaceutically acceptable excipient.

An "effective amount" (or, "therapeutically effective amount") is an amount sufficient to effect a beneficial or desired clinical result upon treatment. An effective amount can be administered to a subject in one or more doses. In terms of treatment, an effective amount is an amount that is sufficient to palliate, ameliorate, stabilize, reverse or slow the progression of the disease, or otherwise reduce the pathological consequences of the disease. The effective amount is generally determined by the physician on a case-by-case basis and is within the skill of one in the art. Several factors are typically considered when determining an appropriate dosage to achieve an effective amount. These factors include age, sex and weight of the subject, the condition being treated, the severity of the condition and the form and effective concentration of the immune cells administered. For adoptive immunotherapy using antigen-specific T cells, cell doses in the range of about 10⁶-10¹⁰ (e.g., about 10⁹) are typically infused. Upon administration of the presently disclosed immune cells into the host and subsequent differentiation, T cells that are specifically directed against the specific antigen expand. The immune cells can be administered by any method known in the art including, but not limited to, intravenous, subcutaneous, intranodal, intratumoral, intrathecal, intrapleural, intraperitoneal and directly to the thymus.

Pharmaceutical compositions comprising the presently disclosed modified immune cells can be conveniently provided as sterile liquid preparations, e.g., isotonic aqueous solutions, suspensions, emulsions, dispersions, or viscous compositions, which may be buffered to a selected pH. Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyol (for example, glycerol, propylene glycol, liquid polyethylene glycol, and the like) and suitable mixtures thereof.

Sterile injectable solutions can be prepared by incorporating the modified immune cells according to the invention in the required amount of the appropriate solvent with various amounts of the other ingredients, as desired. Such compositions may be in admixture with a suitable carrier, diluent, or excipient such as sterile water, physiological saline, glucose, dextrose, or the like. The compositions can also be lyophilized. The compositions can contain auxiliary substances such as wetting, dispersing, or emulsifying agents (e.g., methylcellulose), pH buffering agents, gelling or viscosity enhancing additives, preservatives, flavoring agents, colors, and the like, depending upon the route of administration and the preparation desired. Standard texts, such as "Remington - The Science and Practice of Pharmacy", 23rd edition, 2020, may be consulted to prepare suitable preparations, without undue experimentation.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. Prolonged absorption of the injectable pharmaceutical form can be brought about by the use of agents delaying absorption, for example, aluminum monostearate and gelatin. According to the presently disclosed subject matter, however, any vehicle, diluent, or additive used would have to be compatible with the modified immune cells or their progenitors. The compositions can be isotonic, i.e., they can have the same osmotic pressure as blood and lacrimal fluid. The desired isotonicity of the compositions may be accomplished using sodium chloride, or other pharmaceutically acceptable agents such as dextrose, boric acid, sodium tartrate, propylene glycol or other inorganic or organic solutes. Sodium chloride can be particularly for buffers containing sodium ions.

The skilled artisan can readily determine the amount of cells and optional additives, vehicles, and/or carrier in compositions and to be administered in methods. Typically, any additives (in addition to the active cell(s) and/or agent(s)) are present in an amount of 0.001 to 50% (weight) solution in phosphate buffered saline, and the active ingredient is present in the order of micrograms to milligrams, such as about 0.0001 to about 5 wt %, about 0.0001 to about 1 wt %, about 0.0001 to about 0.05 wt% or about 0.001 to about 20 wt %, about 0.01 to about 10 wt %, or about 0.05 to about 5 wt %. For any composition to be administered to an animal or human, the followings can be determined: toxicity such as by determining the lethal dose (LD) and LD50 in a suitable animal model e.g., rodent such as mouse; the dosage of the composition(s), concentration of components therein and timing of administering the composition(s), which elicit a suitable response. Such determinations do not require undue experimentation from the knowledge of the skilled artisan, this disclosure and the documents cited herein. And, the time for sequential administrations can be ascertained without undue experimentation.

The features, characteristics and advantages disclosed for the isolated modified immune cell are also disclosed for the pharmaceutical composition and apply equally.

Another subject-matter of the disclosure is a method of treating a living being suffering or at risk of suffering from cancer or non-malignant diseases, comprising the administration of the isolated CAR T or NKcell and/or the pharmaceutical composition according to the disclosure.

The quantity of cells to be administered will vary for the subject being treated. In a one embodiment, between about 10⁴ and about 10¹⁰, between about 10⁵ and about 10⁹, or between about 10⁶ and about 10⁸ of the presently disclosed modified immune cells are administered to a human subject. More effective cells may be administered in even smaller numbers. In certain embodiments, at least about 1 x 10⁸, about 2 x 10⁸, about 3 x 10⁸, about 4 x 10⁸, or about 5x 10⁸ of the presently disclosed modified immune cells are administered to a human subject. In certain embodiments, between about 1 x 10⁷ and 5 x 10⁸ of the presently disclosed modified immune cells are administered to a human subject. The precise determination of what would be considered an effective dose may be based on factors individual to each subject, including their size, age, sex, weight, and condition of the particular subject. Dosages can be readily ascertained by those skilled in the art from this disclosure and the knowledge in the art.

In general, and in an embodiment of said method the isolated CAR T or NK cell according to the invention was prepared starting from the living being's own immune cells (autologous) or from immune cells of a reference living being (allogeneic).

The invention is now further explained by means of embodiments resulting in additional features, characteristics and advantages of the invention. The embodiments are of pure illustrative nature.

The invention is now described and explained in further detail by referring to the following non-limiting examples and figures.

### BRIEF DESCRIPTION OF THE FIGURES

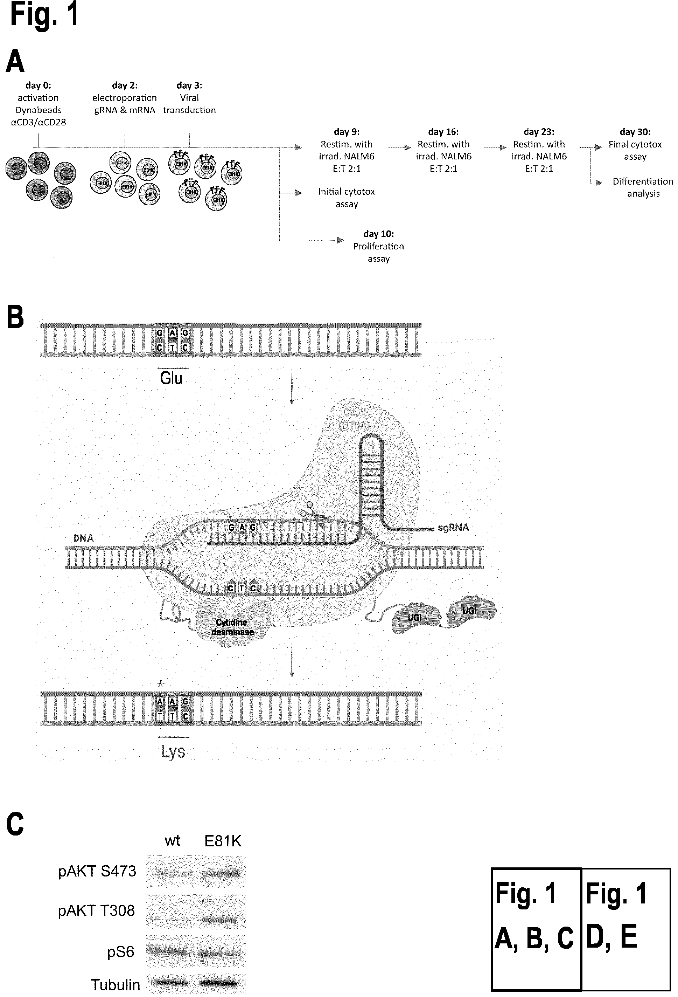

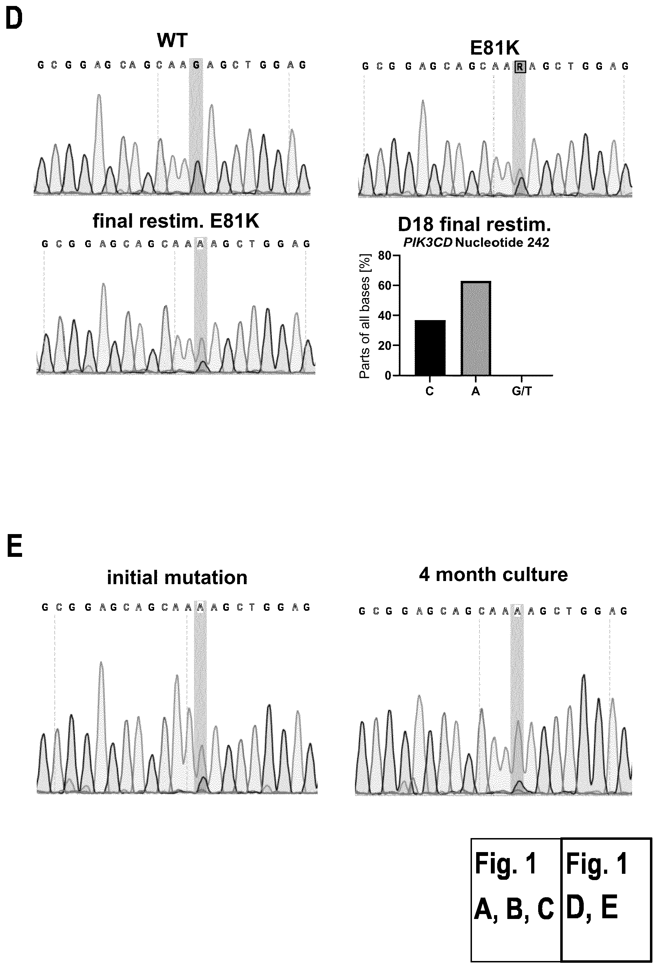

- Fig. 1:: Base editing in CAR T cells. [A] Schematic representation of the generation and experimental testing of base-edited CAR T cells. T cells were isolated from buffy coats and activated using CD3/CD28 beads and the cytokines IL7/IL15 (day 0). Two days later, base editing of T cells was performed by electroporation, and the following day, viral transduction was performed to produce CAR T cells. (Re)stimulation of CAR T cells with target cells was performed at the time points shown using irradiated NALM6 cells (CD19+ B-ALL cell line) in an effector:target ratio of 2:1. At the endpoint, T cell differentiation and cytotoxic activity of CAR T cells were measured. [B] Schematic representation of the base editing technique with the point mutation obtained resulting in a conversion of a cytidine (C) to a thymidine (T) in the PI3KCD gene. The initial editing occurs on the reverse strand, resulting in a guanidine (G) to adenosine (A) mutation on the coding strand. Ultimately, a mutation is present in the PI3Kδ protein, with a lysine (Lys, K) inserted instead of a glutamic acid (Glu, E) at position 81 (E81K). [C] Effect of E81K point mutation on PI3K downstream signaling pathways shown by Western blot using wild-type or E81K-modified SupT1 (T-LBL cell line) single cell clones as examples, tubulin serves as loading control. [D] Sequencing of wild-type control CAR T cells ("WT"), initially edited E81K CAR T cells ("E81K") and edited E81K CAR T cells re-stimulated three times with target cells ("final restim. E81K"). The proportion of the mutated base in the total cell population of the gene PIK3CD at nucleotide position 242 (encoding E81) determined by TIDER (http://shinyapps.datacurators.nl/tider/) is shown after final re-stimulation ("D18 final restim."). The altered base is highlighted in blue. [E] Sequencing of T cells initially and four months after genomic editing. The changed base is highlighted in grey.

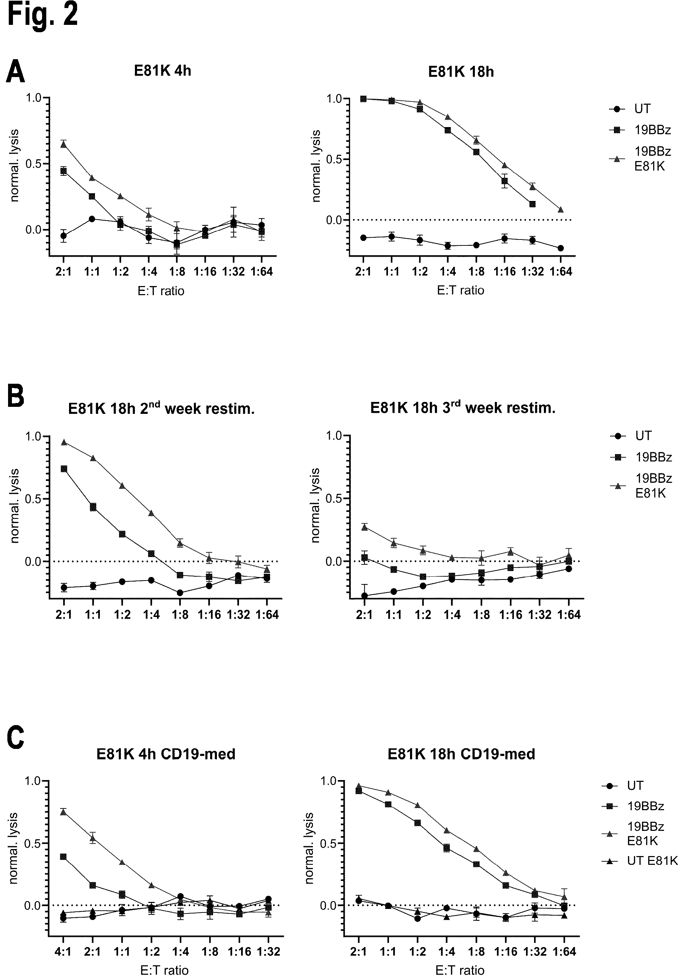

- Fig. 2:: E81K CAR T cells show enhanced cytotoxicity compared to unmodified CAR T cells. [A] Cytotoxic activity of untransduced T cells (UT), 19BBz CAR T cells (19BBz) and E81K-modified 19BBz CAR T cells (19BBz E81K). Luciferase-expressing (ffLuc)-NALM6 cells and CAR T cells were mixed in the ratios shown and cultured for 4h and 18h, respectively. The lysis that occurred was determined by the luminescence signal of the NALM6 cells. [B] Cytotoxic activity of untransduced T cells, 19BBz CAR T cells and 19BBz E81K CAR T cells. CAR T cells after second (left panel) and third re-stimulation (right panel) with the target cells were mixed with ffLuc-NALM6 cells in the ratios shown and cultured for 18h. The lysis that occurred was determined by the luminescence signal of the NALM6 cells. [C] Cytotoxic activity of untransduced T cells (UT), untransduced E81K-modified T cells (UT E81K), 19BBz CAR T cells and 19BBz E81K CAR T cells. ffLuc-NALM6 cells with low CD19 expression (CD19-med) and CAR T cells were mixed in the ratios shown and cultured for 4h and 18h, respectively. The lysis that occurred was determined by the luminescence signal of the NALM6 cells.

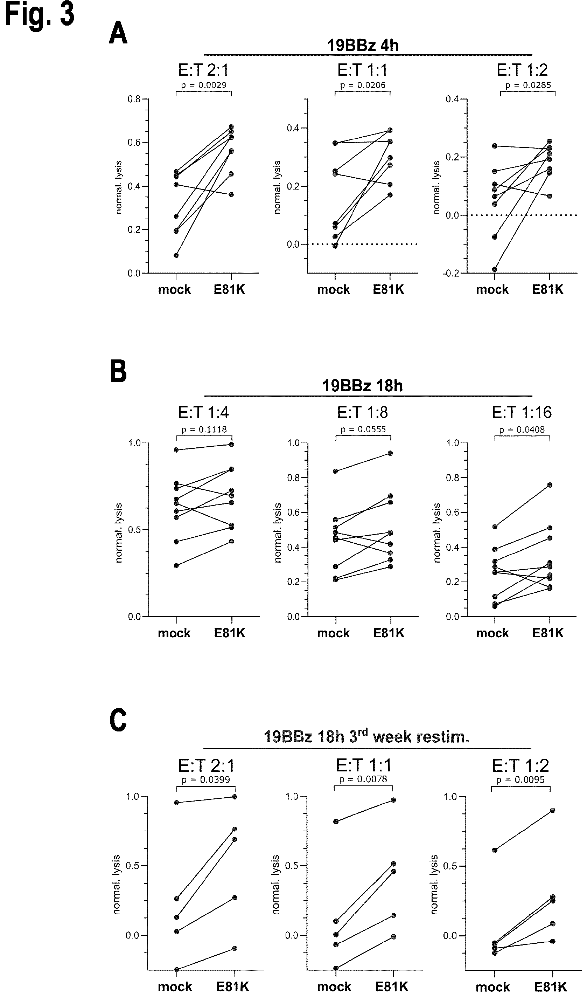

- Fig. 3:: The enhanced cytotoxic activity of CAR T cells by E81K modification persists initially and is maintained after multiple restimulations with tumor cells. [A] Overview of cytotoxic CAR T cell efficacy after 4h co-cultivation of ffLuc-NALM6 cells and 19BBz T cells (mock) or 19BBz E81K T cells (E81K) from healthy donors (n=8). Shown are results at CAR T-cell to NALM6 ratios of 2:1, 1:1 and 1:2. p-values were determined using a paired t-test. [B] Overview of cytotoxic CAR T cell efficacy 18h after co-culturing ffLuc-NALM6 cells and 19BBz T cells or 19BBz E81K T cells from healthy donors (n=8). Shown are results at CAR T-cell to NALM6 ratios of 2:1, 1:1, and 1:2. p-values were determined using a paired t-test. [C] Overview of cytotoxic CAR T cell efficacy 18h after co-culturing ffLuc-NALM6 cells and triply re-stimulated 19BBz T cells or 19BBz E81K T cells from healthy donors (n=5). Shown are results at CAR T cell to NALM6 ratios of 2:1, 1:1, and 1:2. p-values were determined using a paired t-test.

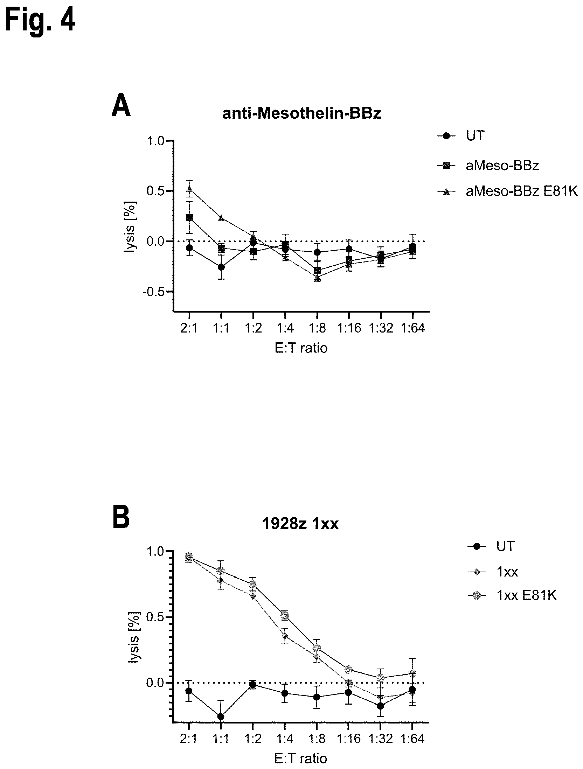

- Fig. 4:: E81K CAR T cells show enhanced cytotoxicity compared to unmodified CAR T cells. [A] Cytotoxic activity of untransduced T cells (UT), anti-Mesothelin-BBz CAR T cells (aMeso-BBz) and E81K-modified anti-Mesothelin-BBz CAR T cells (aMeso-BBz E81K). Luciferase-expressing (ffLuc)-NALM6 cells and CAR T cells were mixed in the ratios shown and cultured for 18h. The lysis that occurred was determined by the luminescence signal of the NALM6 cells. [B] Cytotoxic activity of untransduced T cells (UT), anti-CD19-28z1xx CAR T cells (1xx) and E81K-modified anti-CD19-28z1xx CAR T cells (1xx E81K). Luciferase-expressing (ffLuc)-NALM6 cells and CAR T cells were mixed in the ratios shown and cultured for 18h. The lysis that occurred was determined by the luminescence signal of the NALM6 cells.

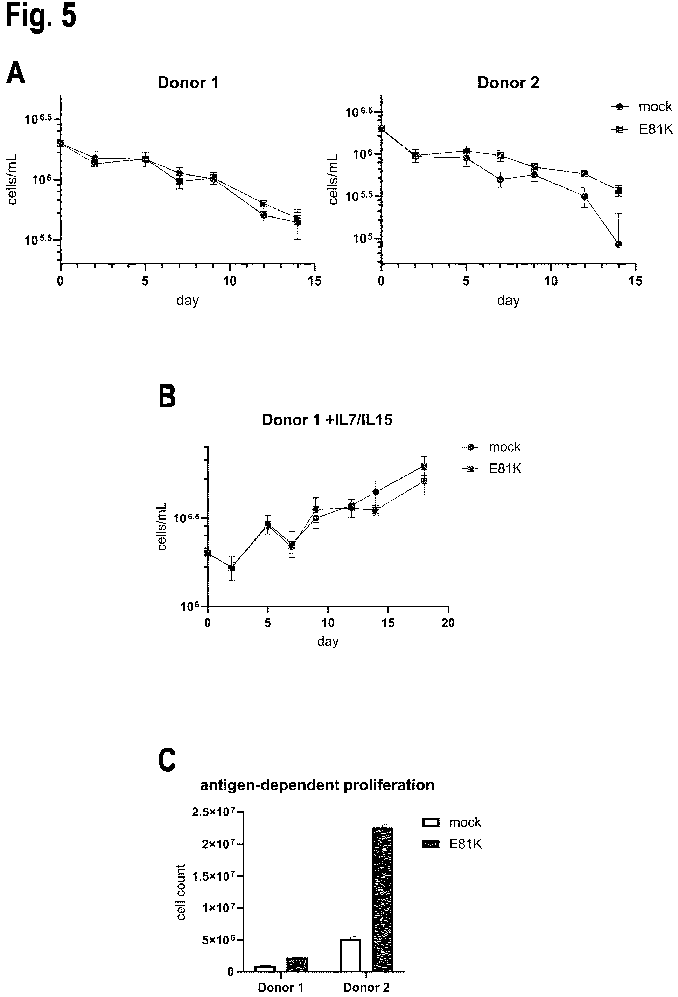

- Fig. 5:: E81K CAR T cells exhibit increased antigen-dependent proliferative capacity but do not show increased antigen-independent proliferative potential. [A] Cell numbers of vital 19BBz CAR T cells with or without E81K point mutation (E81K or mock) from two healthy donors over 14 days after cytokine discontinuation. [B] Cell number of vital 19BBz CAR T cells with and without E81K point mutation under cytokine stimulation using a healthy donor as an example over 18 days. [C] Antigen-dependent proliferation seven days after twice re-stimulation of 19BBz CAR T cells with and without E81K point mutation.

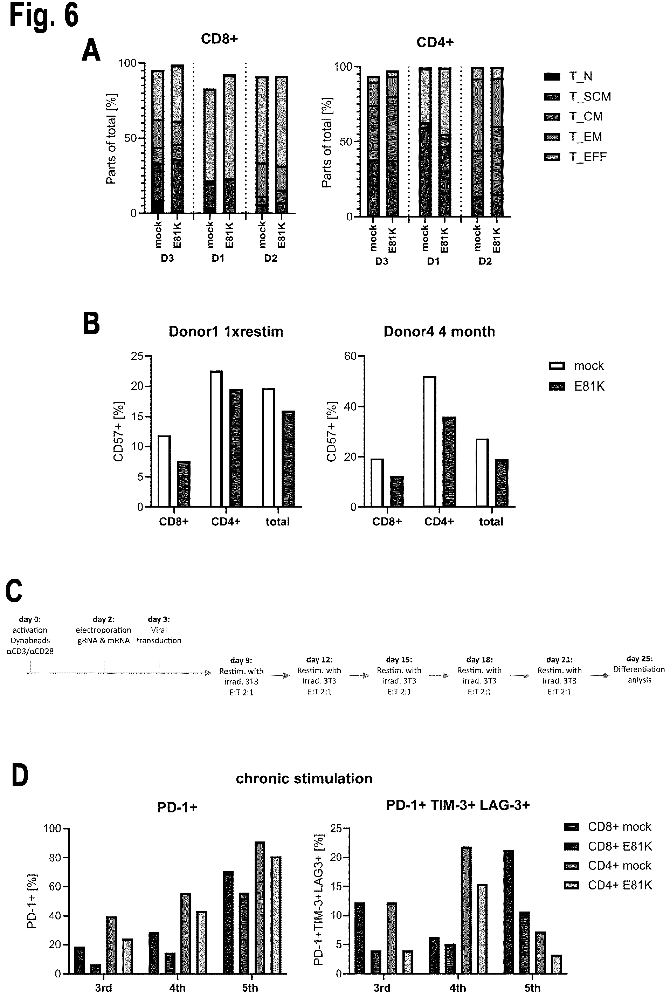

- Fig. 6:: Phenotypic characterization of E81K CAR T cells after chronic antigen stimulation shows no increased T cell differentiation or exhaustion. [A] Differentiation of CD8⁺ and CD4⁺ CAR T cells with (E81K) and without E81K (mock) point mutation after three times re-stimulation with the irradiated B-ALL cell line NALM6 in n=3 donors (D1, D2, D3). Using flow cytometric analysis of surface markers CCR7, CD62L, CD95 and CD45RA to determine T cell differentiation, five different T cell populations are distinguished (naive T cells: T_N; stem cell memory T cells: T_SCM; central memory T cells: T_CM; effector memory T cells: T_EM; effector T cells: T_EFF). [B] The percentage of CD57 positive T cells with (E81K) and without E81K (mock) point mutation was determined by flow cytometry. CAR T cells from donor1 after single restimulation and untransduced T cells from donor4 after four months of cultivation are shown; the results of T cells with (E81K) and without (mock) E81K point mutation are compared. [C] Schematic representation of the generation and experimental evaluation of the phenotype of base-edited CAR T cells after chronic stimulation with target cells compared to controls. Re-stimulation of CAR T cells with (E81K) and without E81K (mock) point mutation were performed at the time points shown with irradiated CD19-overexpressing 3T3 fibroblast cells in an effector to target ratio of 2:1. At the endpoint, CAR T cells were analyzed for PD-1, TIM3, and LAG3 expression by flow cytometry. [D] Proportion of PD-1 positive and PD-1, TIM3 and LAG3 triple positive CD4⁺ and CD8⁺ CAR T cells with and without E81K point mutation after the third, fourth and fifth re-stimulation according to the scheme shown in [C].

### EXAMPLE

### 1. Material and methods

Primary human T cells were isolated from human blood samples and activated for two days with CD3/CD28 Dynabeads in the presence of IL-7 and IL-15. 48h after activation Dynabeads were removed and 3 x 10⁶ T cells were electroporated using the Neon electroporator (1400V, 3pulses, 10ms, Buffer T, 100µl). For each electroporation, 10µg of in vitro synthesized ARCA-capped or CleanCap-capped and PolyA-tailed mRNA of an C to T Base editor like AncBE4max and 10µg of the synthetic gRNA (IDT) were used. 24h after electroporation, cells were transduced with the respective CAR construct using a retroviral system. Cells were cultured under presence of IL-7 and IL-15 until further experiments were performed.

The point mutation was inserted via base editing, a process making use of the CRISPR/Cas9 system. This system relies on a Cas9-nickase which is coupled to a cytidine deaminase, which converts cytosines to uracils, which are read by polymerases as thymines.

Specifically, the catalytically impaired Cas9 nuclease domain localizes a single strand (ss)DNA deaminase enzyme to a genomic target sequence of interest. Upon binding of the Cas9 protein, hybridization of the guide RNA spacer to the target DNA strand causes displacement of the PAM-containing genomic DNA strand to form a ssDNA R-loop. PAM-distal nucleotides are exposed as ssDNA and are accessible to the deaminase domain of the base editor. Therefore, gene editing on a genomic level is achieved by installing a precise point mutation in the respective locus. An additional mutation was not found so far. Nevertheless, a further mutation in the editing window of the specific sgRNA would be possible for Q79, resulting in a silent point mutation.

The AncBE4max Base editor in combination with the following sgRNA sequence "gctcttgctgctccgctgtc" (SEQ ID NO: 1) results in a point mutation in the PIK3CD gene, where the glutamic acid at position 81 gets mutated to a lysine. In general, all C to T Base editors with an NGG or NG PAM can induce this mutation.

The A to G Base editor ABEmax in combination with the following sgRNA sequence "aagagctggaggacgagcaa" (SEQ ID NO: 2) results in a point mutation in the PIK3CD gene, where the glutamic acid at position 81 gets mutated to a glycine. In general, all A to G Base editors with an NGG or NG PAM can induce this mutation.

### 2. Results

Fig. 1A is a schematic representation of the generation and experimental testing of base-edited CAR T cells according to the invention. T cells were isolated from buffy coats and activated using CD3/CD28 beads and the cytokines IL7/IL15 (day 0). Two days later, base editing of T cells was performed by electroporation, and the following day, viral transduction was performed to produce CAR T cells. (Re)stimulation of CAR T cells with target cells was performed at the time points shown using irradiated NALM6 cells (CD19⁺ B-ALL cell line) in an effector:target ratio of 2:1. At the endpoint, T cell differentiation and cytotoxic activity of CAR T cells were measured.

Editing T cells at the genomic level is particularly suitable, as this ensures that there is no exogenous overexpression of PI3Kδ and no uncoupling of the biological receptor, so that negative regulation is also guaranteed. At the same time, however, it also ensures good efficiency and feasibility in primary immune cells.

Fig. 1B is a schematic representation of the base editing technique with the point mutation obtained. Editing at the genomic level is performed by a CRISPR/Cas9 variant, which consists of a Cas9 nickase and causes only a single-strand break. This has the great advantage that the formation of translocations as well as the DNA damage response caused by a possible double strand break is significantly reduced. The Cas9 nickase is also coupled to a cytidine deaminase and two uracil DNA glycosylase inhibitors (UGls), which together constitute the so-called base editor. The editing results in a conversion of a cytidine (C) to a thymidine (T) in the PI3KCD gene. The initial editing occurs on the reverse strand, resulting in a guanidine (G) to adenosine (A) mutation on the coding strand. Ultimately, a mutation is present in the PI3Kδ protein, with a lysine (Lys, K) inserted instead of a glutamic acid (Glu, E) at position 81 (E81K).

Fig. 1C shows the effect of the E81K point mutation on PI3K downstream signaling pathways shown by Western blot using wild-type or E81K-modified SupT1 (T-LBL cell line) single cell clones as examples, tubulin serves as loading control. The application of this method resulted in increased phosphorylation of the downstream protein Akt in the signaling cascade in the T cell line SupT1, indicating increased PI3K activity.

Fig. 1D demonstrates via sequencing of wild-type control CAR T cells ("WT"), initially edited E81K CAR T cells ("E81K") and edited E81K CAR T cells re-stimulated three times with target cells ("final restim. E81K"), that the inserted mutation persists after multiple antigen stimulation in a CAR T-cell context. The proportion of the mutated base in the total cell population of the gene PIK3CD at nucleotide position 242 (encoding E81) determined by TIDER (http://shinyapps.datacurators.nl/tider/) is shown after final re-stimulation ("D18 final restim."). The altered base is highlighted in blue.

As shown in Fig. 1E via the sequencing of T cells initially and four months after genomic editing the inserted mutation is stable in primary T cells for several weeks. The changed base is highlighted in blue.

As shown in Fig. 2A, the E81K mutation in primary 19BBz CAR T cells shows enhanced cytotoxic activity 4 h as well as 18 h after incubation with target cells compared to unmodified CAR T cells.

Even after two- and three-times antigen stimulation, the edited T cells retain the enhanced cytotoxic activity; see Fig. 2B.

Furthermore, the E81K-edited 19BBz CAR T cells showed enhanced cytotoxic activity against tumor cells with lower antigen levels; cf. Fig. 2C.

As shown in Fig. 3A and Fig. 3B, the enhanced cytotoxic activity of E81K-modified CAR T cells after 4h and 18h was significantly different in multiple donors (n=8) and was also maintained after antigen re-stimulation three times; see Fig. 3C. In conclusion, these results demonstrate a sustained increase in the functionality of E81K-modified CAR T cells against tumor cells with different antigen levels.

Enhanced antitumor efficacy of E81K modified CAR T cells could be confirmed with CARs targeting other antigens and against solid tumors (e.g., Mesothelin-specific CARs, Fig. 4A). In addition, the E81K modification leads to an improved therapeutic potential of different CAR designs (e.g., 1928z1XX CAR T cells, Fig. 4B).

To exclude a transforming effect of the E81K mutation, cell growth was examined without (Fig. 5A) and with cytokines (Fig. 5B) as well as with and without antigen stimulation. Thereby, in an antigen-independent context, there was no increased cell proliferation of E81K-mutated CAR T cells compared with wild-type CAR T cells. However, under antigen-stimulation, E81K-mutated CAR T cells showed enhanced cell growth (Fig. 5C), indicating an improved specific proliferation potential of E81K-mutated CAR T cells.

Re-stimulation with antigen-expressing target cells three times resulted in a slightly increased proportion of effector T cells in the E81K-mutated 19BBz CAR T cells, but without showing a significantly altered differentiation pattern; see Fig. 6A. Moreover, the E81K mutation in T cells and CAR T cells did not result in increased expression of CD57, which is known as a marker for senescent T cells; see Fig. 6B. Moreover, even under extensive chronic antigen stimulation (Fig. 6C), the E81K mutant CAR T cells did not show enhanced expression of inhibitory surface markers such as PD-1, TIM3, and LAG3, which are associated with T cell exhaustion and CAR T cell dysfunction; see Fig. 6D.

In summary, the results show that E81K mutation induced by base editing leads to a sustained increase in CAR T cell functionality. E81K mutated CAR T cells exhibit increased cytotoxicity and antigen-dependent proliferation, indicating improved therapeutic potential compared to currently approved CAR T cell products. The induced mutation is stably maintained for several weeks. A PIK3CD E81K mutation has been described in a few patients, where it resulted in an increased proportion of CD57⁺ T cells and an increased induction of T cell exhaustion during the course. The inventors could not observe this development in the CAR T-cell setting even after multiple antigen stimulation of the CARs, but could demonstrate a sustained increased functionality of the E81K mutated CAR T cells.

On the one hand, it is significant for the analysis of the safety of the cell product that the E81K mutated T cells do not show an increased antigen-independent proliferation potential, so that no increased transformation potential can be assumed (Fig. 4A and B). Furthermore, the E81K mutation has been detected in tumor patients in only four cases out of a cohort of over 64,000 cases, and no T cell tumor is represented (https://www.cbioportal.org/). This suggests that the E81K mutation in T cells is not associated with malignant potential. This is also confirmed by the fact that patients with E81K mutations did not develop T-cell malignancy in the two cases described above.

A limitation of current CAR T cell products is also the reduced sensitivity to tumor cells with low antigen expression - this can lead to recurrences of tumor cells with reduced antigen density and is also relevant in solid tumors given their heterogeneity. The functional enhancement of CAR T cells by the introduced E81K mutation is also confirmed against tumor cells with low antigen expression, so that the therapeutic potential of CAR therapy could also be improved in terms of enhanced antigen sensitivity.

Overall, the results show that an E81K mutation can be successfully generated in CAR T cells by genetic modification, leading to a sustained increase in functionality against tumor cells with different antigen expression levels. Notably, the increased activation does not lead to increased T cell exhaustion or senescence. Mutations in the PI3K pathway and in particular E81K mutations thus have great potential to further improve CAR therapies and prevent recurrences. Furthermore, this gene editing strategy can be extended to other cellular therapies, used in combination therapies and thus evaluated as a therapy for tumor diseases and other severe diseases.

## Claims

1. A chimeric antigen receptor (CAR) T or NK cell **characterized by** a modified phosphoinositide 3-kinase (PI3K) pathway compared to a non-modified reference immune cell, which CAR T or NK cell comprises a modified PI3K **characterized by** increased activity compared to a wild type PI3K, wherein said modified PI3K comprises a point mutation.

2. The CAR T or NK cell of claim 1, **characterized by** an overactive PI3K pathway compared to a non-modified reference immune cell.

3. The CAR T or NK cell according to claim 1 or 2, **characterized in that** said modified PI3K comprises the point mutation at amino acid position 81.

4. The CAR T or NK cell according to claim 3, **characterized in that** by said point mutation in PI3K a glutamic acid (E) is replaced by a lysine (K) (PI3K^{E81K}).

5. The CAR T or NK cell according to any of the preceding claims, which CAR comprises an extracellular antigen-binding domain, a transmembrane domain, and an intracellular signaling domain.

6. The isolated modified immune cell according to claim 5, **characterized in that** the intracellular signaling domain comprises a CD3ζ polypeptide.

7. The modified immune cell according to claims 5 or 6, **characterized in that** the intracellular signaling domain comprises a CD137 (4-1BB) or CD 28 costimulatory polypeptide.

8. The modified immune cell according to any of claims 5-7, **characterized in that** the extracellular antigen-binding domain comprises a CD19 binding polypeptide.

9. The modified immune cell according to any of claims 2-8, **characterized in that** the PI3K is modified by genome editing, preferably by CRISPR/Cas editing.

10. A method for preparing a chimeric antigen receptor (CAR) T or NK cell **characterized by** a modified phosphoinositide 3-kinase (PI3K) pathway compared to a non-modified reference immune cell, comprising (i) providing a CAR T or NK cell, and (ii) introducing a point mutation into PI3K comprised by the CAR T or NK cell such that its activity is increased compared to wild type PI3K.

11. The method of claim 10, **characterized in that** in step (ii) the PI3K is modified by genome editing, preferably by CRISPR/Cas9 editing.

12. The method of claim 11, **characterized in that** a sgRNA molecule is used comprising the nucleotide sequence of gctcttgctgctccgctgtc (SEQ ID NO: 1) and/or aagagctggaggacgagcaa (SEQ ID NO: 2).

13. The modified immune cell according to any of claims 1-9 for use in medicine, preferably for prophylaxis and/or treatment of cancer, further preferably leukemias and/or lymphomas.

## Patentansprüche

1. Chimäre Antigenrezeptor (CAR) -T- oder -NK-Zelle, **gekennzeichnet durch** einen im Vergleich zu einer nicht modifizierten Referenz-Immunzelle veränderten Phosphoinositid-3-Kinase (PI3K) -Signalweg, wobei die CAR-T- oder -NK-Zelle eine modifizierte PI3K mit im Vergleich zu einer Wildtyp-Pl3K erhöhter Aktivität aufweist, wobei die modifizierte PI3K eine Punktmutation aufweist.

2. CAR-T- oder -NK-Zelle nach Anspruch 1, **gekennzeichnet durch** einen im Vergleich zu einer nicht modifizierten Referenz-Immunzelle überaktiven PI3K-Signalweg.

3. CAR-T- oder -NK-Zelle nach Anspruch 1 oder 2, **dadurch gekennzeichnet, dass** die modifizierte PI3K die Punktmutation an der Aminosäureposition 81 aufweist.

4. CAR-T- oder -NK-Zelle nach Anspruch 3, **dadurch gekennzeichnet, dass** durch die Punktmutation in PI3K eine Glutaminsäure (E) durch ein Lysin (K) ersetzt ist (PI3K^{E81K}).

5. CAR-T- oder -NK-Zelle nach einem der vorhergehenden Ansprüche, wobei der CAR einen extrazellulären antigenbindenden Bereich, einen Transmembranbereich und einen intrazellulären Signalbereich aufweist.

6. Isolierte modifizierte Immunzelle nach Anspruch 5, **dadurch gekennzeichnet, dass** der intrazelluläre Signalbereich ein CD3ζ-Polypeptid aufweist.

7. Modifizierte Immunzelle nach Anspruch 5 oder 6, **dadurch gekennzeichnet, dass** der intrazelluläre Signalbereich ein CD137(4-1BB)- oder CD28-kostimulatorisches Polypeptid aufweist.

8. Modifizierte Immunzelle nach einem der Ansprüche 5 bis 7, **dadurch gekennzeichnet, dass** der extrazelluläre antigenbindende Bereich ein CD19-bindendes Polypeptid aufweist.

9. Modifizierte Immunzelle nach einem der Ansprüche 2 bis 8, **dadurch gekennzeichnet, dass** die PI3K durch Genom-Editierung, vorzugsweise durch CRISPR/Cas-Editierung, modifiziert ist.

10. Verfahren zur Herstellung einer chimären Antigenrezeptor(CAR)-T- oder -NK-Zelle, **gekennzeichnet durch** einen im Vergleich zu einer nicht modifizierten Referenz-Immunzelle veränderten Phosphoinositid-3-Kinase(PI3K)-Signalweg, das Folgendes aufweist, nämlich (i) die Bereitstellung einer CAR-T- oder -NK-Zelle, und (ii) die Einführung einer Punktmutation in die von der CAR-T- oder -NK-Zelle aufweisende PI3K, sodass deren Aktivität im Vergleich zur Wildtyp-Pl3K erhöht ist.

11. Verfahren nach Anspruch 10, **dadurch gekennzeichnet, dass** in Schritt (ii) die PI3K durch Genom-Editierung, vorzugsweise durch CRISPR/Cas9-Editierung, modifiziert wird.

12. Verfahren nach Anspruch 11, **dadurch gekennzeichnet, dass** ein sgRNA-Molekül mit der Nukleotidsequenz gctcttgctgctccgctgtc (SEQ ID Nr.: 1) und/oder aagagctggaggacgagcaa (SEQ ID Nr.: 2) verwendet wird.

13. Modifizierte Immunzelle nach einem der Ansprüche 1 bis 9 zur Verwendung in der Medizin, vorzugsweise zur Prophylaxe und/oder Behandlung von Krebs, besonders bevorzugt von Leukämien und/oder Lymphomen.

## Revendications

1. Cellule T ou NK à récepteur antigénique chimérique (CAR) **caractérisée par** une voie de la phosphoinositide 3-kinase (PI3K) modifiée par rapport à une cellule immunitaire de référence non modifiée, laquelle cellule CAR T ou NK comprend une PI3K modifiée **caractérisée par** une activité augmentée par rapport à une PI3K de type sauvage, dans laquelle ladite PI3K modifiée comprend une mutation ponctuelle.

2. Cellule CAR T ou NK selon la revendication 1, **caractérisée par** une voie PI3K hyperactive par rapport à une cellule immunitaire de référence non modifiée.

3. Cellule CAR T ou NK selon la revendication 1 ou 2, **caractérisée en ce que** ladite PI3K modifiée comprend la mutation ponctuelle à une position 81 d'acide aminé.

4. Cellule CAR T ou NK selon la revendication 3, **caractérisée en ce que**, par ladite mutation ponctuelle dans la PI3K, un acide glutamique (E) est remplacé par une lysine (K) (PI3K^{E81K}).

5. Cellule CAR T ou NK selon l'une des revendications précédentes, lequel CAR comprend un domaine de liaison à l'antigène extracellulaire, un domaine transmembranaire et un domaine de signalisation intracellulaire.

6. Cellule immunitaire modifiée isolée selon la revendication 5, **caractérisée en ce que** le domaine de signalisation intracellulaire comprend un polypeptide CD3ζ.

7. Cellule immunitaire modifiée selon la revendication 5 ou 6, **caractérisée en ce que** le domaine de signalisation intracellulaire comprend un polypeptide co-stimulateur CD137 (4-1BB) ou CD 28.

8. Cellule immunitaire modifiée selon l'une des revendications 5 à 7, **caractérisée en ce que** le domaine de liaison à l'antigène extracellulaire comprend un polypeptide de liaison à CD19.

9. Cellule immunitaire modifiée selon l'une des revendications 2 à 8, **caractérisée en ce que** la PI3K est modifiée par édition du génome, de préférence par édition CRISPR/Cas.

10. Procédé de préparation d'une cellule T ou NK à récepteur antigénique chimérique (CAR) **caractérisée par** une voie de la phosphoinositide 3-kinase (PI3K) modifiée par rapport à une cellule immunitaire de référence non modifiée, comprenant (i) la fourniture d'une cellule CAR T ou NK, et (ii) l'introduction d'une mutation ponctuelle dans la PI3K comprise dans la cellule CAR T ou NK de sorte que son activité soit augmentée par rapport à la PI3K de type sauvage.

11. Procédé selon la revendication 10, **caractérisé en ce que**, dans l'étape (ii), la PI3K est modifiée par édition du génome, de préférence par édition CRISPR/Cas9.

12. Procédé selon la revendication 11, **caractérisé en ce qu'**une molécule d'ARNsg est utilisée comprenant la séquence nucléotidique de gctcttgctgctccgctgtc (SEQ ID NO : 1) et/ou aagagctggaggacgagcaa (SEQ ID NO : 2).

13. Cellule immunitaire modifiée selon l'une des revendications 1 à 9 pour une utilisation en médecine, de préférence pour la prophylaxie et/ou le traitement du cancer, en outre de préférence des leucémies et/ou des lymphomes.
